Europäisches Patentamt

European Patent Office

Office européen des brevets

(19)

(11) Publication number: **0 233 645**
**A2**

# (12) EUROPEAN PATENT APPLICATION

(21) Application number: **87102312.3**

(22) Date of filing: **18.02.87**

(51) Int. Cl.⁴: **C07K 7/10 , C12P 21/02**

(30) Priority: **18.02.86 US 830420**

(43) Date of publication of application:
**26.08.87 Bulletin 87/35**

(84) Designated Contracting States:
**AT BE CH DE FR GB IT LI LU NL SE**

(71) Applicant: **PHILLIPS PETROLEUM COMPANY**
**5th and Keeler**
**Bartlesville Oklahoma 74004(US)**

(72) Inventor: **Craig, William Scot**
**6094 Via Regla**
**San Diego, CA 92122(US)**
Inventor: **Wondrack, Lillian Margaret**
**1527 Grand Avenue**
**San Diego, CA 92109(US)**

(74) Representative: **Dost, Wolfgang, Dr. rer. nat.**
**Dipl.-Chem. et al**
**Patent- u. Rechtsanwälte Bardehle,**
**Pagenberg, Dost, Altenburg Frohwitter &**
**Partner Galileiplatz 1**
**D-8000 München 80(DE)**

(54) **Purification of growth hormone releasing factor from yeast strains producing same.**

(57) A method for purifying secreted, recombinant growth hormone releasing factor from yeast cultures transformed with vectors that code for the growth hormone releasing factor peptide is disclosed. A sequence of steps is involved, including preparation of a cell-free broth from the fermentation liquor, pH adjustment, initial broth concentration employing reverse phase resins, size fractionation of the initially concentrated material, and finally fractionation of the partially purified material employing high pressure liquid chromatography.

## PURIFICATION OF GROWTH HORMONE RELEASING FACTOR FROM YEAST STRAINS PRODUCING SAME

This invention relates to the isolation and recovery of growth hormone releasing factor (GRF) produced by genetically modified yeast organisms.

### BACKGROUND

Several important hormones are produced in the mammalian hypothalamus and in the anterior lobe of the pituitary gland. One such important hormone is growth hormone (GH), so designated because it promotes mammalian growth. It has been established that release of growth hormone by the pituitary is subject to regulation by hypothalamic peptides. It has been known for some time that a hypothalamic peptide, somatostatin, inhibits release of GH. The concept has been well established that a substance produced in the hypothalamus, referred to as growth hormone releasing factor (GRF) or somatocrinin, promotes the release of GH from the pituitary.

Although GRF is generally associated with the hypothalamus, it may be produced by other cells, such as pancreatic tumor cells. Pancreatic GRF is highly effective in promoting release of growth hormone, and in view of its potential for regulating animal or human growth, it would be highly desirable to be able to obtain human pancreatic GRF in substantial quantities.

Native GRF has 44 amino acid residues and is amidated at its carboxyl end. A form of GRF having only 40 amino acid residues has also been reported, and fragments of GRF having as few as about 27 amino acid residues have been found to be biologically active, although less than the native 44 amino acid residue peptide. The full 44 amino acid residue pancreatic GRF peptide has been synthesized chemically, and synthetic GRF, synthetic GRF fragments, and synthetic analogs thereof represent a potential source of highly potent regulatory substances; however, chemical synthesis of such long peptide chains is quite expensive.

Recombinant DNA techniques by which DNA that codes for peptides having GRF activity is introduced into a cell to express GRF or GRF analogs is believed to represent the best hope of inexpensively producing GRF. By transforming appropriate host organisms with DNA which encodes the GRF amino acid sequence, genetically modified signal cell organisms can be employed to produce GRF in significant quantities. One barrier to employing recombinant DNA technology for the commercial production of GRF is the need to be able to efficiently recover GRF in biologically active form from genetically modified organisms which produce GRF.

### OBJECTS OF THE INVENTION

An object of the present invention, therefore, is to provide a method for the efficient recovery of biologically active GRF produced by genetically modified yeast organisms.

This and other objects of the present invention will become apparent from inspection of the disclosure and claims herein provided.

### STATEMENT OF THE INVENTION

In accordance with the present invention, there has been developed a method for the isolation and purification of biologically active growth hormone releasing factor (GRF) produced by a genetically modified yeast organism, and secreted into the growth medium of the host yeast cell, which method comprises:

(a) separating the cells and cell debris from the fermentation liquor,

(b) adjusting the pH of the cell-free product of step (a) to within the range of about 2 up to 4,

(c) contacting the product of step (b) under adsorption conditions with a reverse phase resin which adsorbs the GRF in the liquor,

(d) eluting the adsorbed GRF from said reverse phase resin,

(e) concentrating the eluate obtained from step (d) to provide a GRF concentrate,

(f) fractionating the product of step (e) to remove substantially all components having molecular weights above about 15,000 and below about 2,000, and

(g) fractionating the product of step (f) by high performance liquid chromatography, and thereafter,

(h) recovering the substantially pure GRF containing fractions.

### DETAILED DESCRIPTION OF THE INVENTION

The present invention involves the isolation and purification of secreted, biologically active GRF produced by a genetically modified yeast organism. Any yeast organism capable of producing and secreting protein products is useful in the practice of the present invention. Presently preferred are strains of *Saccharomyces cerevisiae*, because regulatory sequences and secretion signal sequences operative in such host organisms have been identified and are readily available to those of skill in the art.

The production of GRF by growth of genetically modified organisms has been previously described. Those of skill in the art are directed to disclosures such as EPA 108,387 for greater detail as to the production of GRF by genetically modified organisms.

The first step in the inventive purification process is to separate the cells and cell debris from the fermentation liquor. Any suitable means selected from the various techniques known by those of skill in the art can be employed for such purpose. For example, techniques such as centrifugation, tangential filtration, and the like can be employed for the preparation of the desired cell-free fermentor broth.

The pH of the cell-free fermentor broth is then adjusted to within the range of about 2 up to 4, employing appropriate acids as are readily known by those of skill in the art. For example, initial pH adjustment can be carried out employing acetic acid, with a stronger acid such as hydrochloric acid being employed to reach the finally desired pH.

Where it is necessary to store the GRF-containing fermentor broth, it is desirable to store the broth as the acidified, clarified broth prepared as described hereinabove. If the temperature of storage is maintained in the range of that found in a standard cold room, i.e., about 3 up to 6°C, the desired GRF product contained in the broth will remain stable for extended periods of at least about one month.

The GRF in the acidified, cell-free broth prepared as described above is then adsorbed on a reverse phase resin, then eluted from the resin.

Suitable reverse phase resins include, but are not limited to, C8-C18 resins, CN (cyano), NH$_2$ - (amino) resins, phenyl resins and the like. Those of skill in the art recognize that numerous reverse phase resins are available for use in the practice of the present invention, such as for example, VyC18-20-1, SYC8-20-1, VyC18-5-1, etc., available from Western Analytical Products Company, Inc. of Tenecula, California.

The quantity of resin employed in this adsorption step will vary as a function of the original GRF concentration in the broth, the quantity of broth being treated, the particular reverse phase resin being employed, etc. One of skill in the art, knowing approximate values for the variables recited above, can readily determine approximate quantities of resin to employ. As a minimum, sufficient resin should be employed to ensure that substantially all of the GRF in the original broth is adsorbed.

Following the adsorption step, the resin can optionally be washed with a non-eluting buffer to remove any lightly bound material from the reverse phase resin. The GRF is then eluted from the reverse phase resin employing a minimum volume of an eluting buffer, e.g., varying percentage mixtures of a non-eluting buffer and a polar organic solvent such as, for example, 2-propanol or acetonitrile.

Once eluted from the reverse phase resin, the GRF-eluting buffer solution is concentrated by removing sufficient solvent so that the GRF concentration at this stage of the purification is at least 100 times greater than the GRF concentration in the original broth. Such concentration can be accomplished by various means as well known by those of skill in the art, such as, for example, reduced pressure evaporation employing a rotary evaporator, or the like.

Optionally, the concentrated GRF can be clarified of particulate matter at this point by subjecting the concentrated GRF to a short period of centrifugation.

The concentrated, partially purified GRF is then fractionated to remove molecules having molecular weights in excess of about 15,000 as well as molecules having molecular weights below about 2000. This is most readily accomplished by passing the GRF-containing solutions over separate beds of size exclusion resins having appropriate molecular weight cutoffs.

The fractionated material can optionally be concentrated, as described above, or can be subjected directly to high performance liquid chromatography (HPLC) employing a reverse phase resin, whereby the GRF is first adsorbed on the HPLC column, then the adsorbed GRF is recovered by elution with an eluting buffer, i.e., a solvent system of appropriate polarity to cause the GRF to desorb from the resin.

The present invention will now be described in greater detail by reference to the following nonlimiting example.

EXAMPLE

This example describes the purification of secreted, recombinant growth hormone releasing factor (recGRF) from cultures of *Saccharomyces cerevisiae* transformed with vectors containing sequences that code for production and secretion of GRF peptides. Cultures of *Saccharomyces cerevisiae*, e.g., *S. cerevisiae* pTRP213, were grown in a 10 liter fermentor to a cell density of approximately 20 optical density units (measured at 600 nm). GRF levels, monitored with a radioimmune assay (RIA), were typically in the range 5-30 mg $L^{-1}$. After the cells and cell debris were removed by tangential filtration (Pellicon Apparatus, 0.45µ filter; Millipore Corp.), the pH of the effluent was adjusted with acid to a value of 2, and then stored at 3°C until further treatment.

To concentrate the recGRF from the cell-free media, the effluent was adsorbed onto a reverse phase resin. Following brief equilibration to ambient temperature, the acidified, cell-free media was pumped through a column of octadodecyl silane (C18) reverse phase resin (5 g of Vydac resin in a 2 cm x 2 cm column) previously equilibrated in 0.25 M triethylammonium formate (TEAF), pH = 3.0. The flow rate was kept low (5 mL/min) in order to maximize the binding of recGRF to the resin (less than 1% unbound). This low flow rate resulted in a processing rate of only 4 liters in 12 hours employing 5 g of resin, or about 15-20 volumes of broth per gram of resin per hour. After a wash with TEAF buffer, the bound recGRF radioimmune activity was eluted from the resin with a 50:50 (v/v) mixture of TEAF and isopropanol (20 mL per 5 g resin). The volume of eluate was reduced to near dryness by rotary evaporation.

A variety of yeast metabolites were concentrated with the recGRF and must be removed prior to high performance liquid chromatography - (HPLC) purification of the peptide products. The contaminating metabolites were removed by passage of the concentrated eluate through a column of size exclusion resins. The column (4 cm x 24 cm), equilibrated in 0.5 $\underline{N}$ acetic acid, was composed of Sephadex G-50 (bottom 50%) overlayed with Sephadex G-25 (upper 50%). Approximately 90% of the RIA active material from the column was pooled for further separation using HPLC.

The fraction of recGRF containing the radioimmune activity from the size exclusion column were loaded directly onto a preparative scale (250 mm x 25.4 mm) C8 HPLC column previously equilibrated with a 75:25 (v/v) mixture of 0.25 $\underline{M}$ triethylammonium phosphate (TEAP), pH 3.0 and acetonitrile. The recGRF peptides, detected by adsorption at 210 nm, were eluted with a linear gradient from 25% to 40% acetonitrile.

If impurities which interfere with the optical detection of peptides during this HPLC step remain in the sample at this point, the following additional treatment step can be employed. The original eluate, at pH6, can be applied to a small column - (~1 gram) of hydroxyapatite (HAP) resin, and then passed over the gel filtration columns again.

The fractions containing biological activity, as detected in the *in vitro* growth hormone releasing assay (described below), were pooled. After removal of the organic reagent by evaporation, the sample was injected onto a semi-preparative scale (250 mm x 10 mm) C18 HPLC column previously equilibrated with a 75:25 (v/v) mixture of 0.05% aqueous trifluoroacetic acid (TFA) and 0.05% TFA in acetonitrile. The recGRF peptides were eluted with a linear gradient from 25% to 50% acetonitrile. The organic reagent was removed by evaporation and the TFA by lyophilization.

The recGRF peptide obtained using the above described procedure possessed *in vitro* biological activity that was indistinguishable from the activity of a synthetic peptide standard. Thus, analysis using discontinuous polyacrylamide gel electrophoresis under denaturing conditions demonstrated less than 5% contamination from other peptides. In addition, dansylation of the peptide yielded a single residue, tyrosine, as the N-terminal amino acid. Therefore, the isolation and purification procedure herein disclosed provides an effective means for recovery of highly purified GRF from recombinant organisms.

The *in vitro* growth hormone releasing factor assay employed in the course of this work was carried out as follows. The yeast-produced GRF was tested for its ability to stimulate the release of GH from rat pituitary cells in culture. The standard used for comparison was hpGRF (1-40)OH. This peptide elicits a 3-5-fold maximal stimulation in GH secretion above the $EC_{50}$ (effective concentration that causes a 50% increase in secretion).

## Methods

Preparation of Primary Cultures of Rat Anterior Pituitaries: Rat anterior pituitaries were removed from Sprague-Dawley rats (200-225 g) that has been sacrificed with rapid decapitation. The procedure used for dissociation into cells involves, briefly, halving the anterior lobes and incubating for 2 hours at 37°C in a spinner suspension flask in HDB (25 mM Hepes, 137 mM NaCl, 5 mM KCl, 0.7 mM $Na_2HPO_4$, 10 mM glucose, pH 7.3) containing 0.4% collagenase and 80 µg/mL DNase-II (one mL solution per pituitary). Subsequently, the cells were washed three times in B-pit julep medium containing 2% fetal calf serum, Sato's cocktail, and 5 nM dexamethasone. Following the final wash, an aliquot was counted and the cells were suspended in a final concentration of 3.3 X 105 cells/mL and plated in 24-well Linbro tissue culture plates (Flow Labs). The plates were placed in a water-jacketed incubator maintained at 37°C and 5% CO2. Approximate yields were 2 X $10^6$ cells per pituitary.

Treatments for Bioassays: Cells were generally assayed for biological activity, i.e., GH secretion, within 3-5 days of initial plating. At this time, the cells were washed three times with B-pit julep medium containing 1 mg/mL crystalline BSA - (Miles) and 50 ug/mL ascorbic acid. The cells were normalized in this serum free medium by one hour preincubation prior to the particular treatments. The treatments were generally added in 100uL of 10X the final desired dose to wells containing 900uL of the medium. Treatments were carried out for 4 hours at 37°C, and subsequently, the media were removed from each well and tested for GH levels. Furthermore, the cells were permeabilized by incubation with 0.1% Triton X-100 for 15 minutes at 37°C in order to measure total cellular GH content. At the end of 15 minutes, the wells were washed with 3mL of medium and again the GH levels were measured by RIA.

The example has been provided merely to illustrate the practice of the invention and should not be read so as to limit the scope of the invention or the appended claims in any way. Reasonable variations and modifications, not departing from the essence and spirit of the invention, are contemplated to be within the scope of patent protection desired and sought.

## Claims

1. A method for the purification of secreted growth hormone releasing factor (GRF) produced by growth of recombinant yeast organisms, **characterized by**

(a) separating the cells and cell debris from the fermentation liquor,

(b) adjusting the pH of the cell-free product of step (a) to within the range of 2 to 4,

(c) contacting the product of step (b) under adsorption conditions with a reverse phase resin which adsorbs the GRF in the liquor,

(d) eluting the adsorbed GRF from said reverse phase resin,

(e) concentrating the eluate obtained from step (d) to provide a GRF concentrate in the range of 100 to 10,000 times the original concentration of GRF in the fermentation liquor,

(f) fractionating the product concentrate of step (e) to remove substantially all components having molecular weights above about 15,000 and below about 2,000, and

(g) fractionating the product of step (f) by high performance liquid chromatography, and thereafter

(h) recovering the substantially pure GRF containing fractions.

2. The method of claim 1, characterized in that said recombinant yeast organism is a strain of Saccharomyces cerevisiae, preferably Saccharomyces cerevisiae pTRP213.

3. The method of claim 1 or 2, characterized in that step (a) is accomplished by tangential filtration.

4. The method of any of the preceding claims, characterized in that the step (b) pH adjustment is accomplished with at least one acid selected from acetic acid and hydrochloric acid.

5. The method of any of the preceding claims, characterized in that said reverse phase resin employed in step (c) is a C18 resin, in particular wherein the pH adjusted, cell-free product of step - (b) is passed over the reverse phase resin at the rate of 15 to 20 volumes of said product per gram of resin per hour.

6. The method of any of the preceding claims, characterized in that after step (c) but before step - (d), the reverse phase resin having GRF adsorbed thereon is washed with at least one volume of non-eluting buffer.

7. The method of any of the preceding claims, characterized in that said eluting solvent system comprises a 50:50 (v/v) mixture of 0.25M triethylamine formate, pH=3.0, and isopropanol; in particular wherein about 4 volumes of eluting solvent system are employed per gram of resin.

8. The method of any of the preceding claims, characterized in that after step (f), but before step - (g), the product of step (f) is contacted with hydroxyapatite resin and the step (f) is repeated before carrying out step (g).

9. The method of any of the preceding claims, characterized in that step (g) comprises two passes over a C18 resin; in particular wherein the adsorbed GRF is eluted from the first pass with a solvent system comprising a linear gradient of 25 to 40% acetonitrile in 0.25M triethylammonium phosphate, pH 3.0; and the adsorbed GRF is eluted from the second pass with a solvent system comprising a linear gradient of 25 to 50% acetonitrile in 0.05% aqueous trifluoroacetic acid.

10. The method of any of the preceding claims, characterized in that purified, solvent free GRF is recovered from the GRF-containing fractions by evaporation of the acetonitrile and lyophilization of the trifluoroacetic acid.